# EUROPÄISCHE PATENTSCHRIFT

(11) **EP 3 356 816 B1**
(45) Veröffentlichungstag und Bekanntmachung des Hinweises auf die Patenterteilung: **09.11.2022**
(21) Anmeldenummer: 16766905.0
(22) Anmeldetag: 12.09.2016
(51) Int. Cl.: G01N 33/44

(54) **VERFAHREN ZUR BESTIMMUNG EINES MISCHUNGSVERHÄLTNISSES VON ZWEI ORGANISCHEN, MISCHBAREN KOMPONENTEN IN EINEM GEMISCH DIESER KOMPONENTEN**
METHOD FOR DETERMINING A MIXING RATIO OF TWO ORGANIC, MISCIBLE COMPONENTS IN A MIXTURE OF SAID COMPONENTS
PROCÉDÉ DE DÉTERMINATION D'UN RAPPORT DE MÉLANGE DE DEUX COMPOSANTS ORGANIQUES POUVANT ÊTRE MÉLANGÉS DANS UN MÉLANGE DE CES COMPOSANTS

(30) Priorität: 28.09.2015 DE 102015218600
(43) Veröffentlichungstag der Anmeldung: 08.08.2018
(73) Patentinhaber: Bayerische Motoren Werke Aktiengesellschaft, 80809 München (DE)
(72) Erfinder: LÄSSIG, Daniel, 04435 Schkeuditz (DE); HESSING, Florian, 33100 Paderborn (DE); NIKOLEIZIG, Ines, 06242 Braunsbedra (DE)
(86) Internationale Anmeldenummer: PCT/EP2016/071439
(87) Internationale Veröffentlichungsnummer: WO 2017/055066

(56) Entgegenhaltungen:
- EP-A1- 2 314 442
- WO-A1-2013/091623
- DE-A1- 4 434 815
- US-A- 4 478 941

## Beschreibung

Die vorliegende Erfindung betrifft ein Verfahren zur Bestimmung eines Mischungsverhältnisses einer Anzahl von zwei organischen, mischbaren Komponenten in einem Gemisch dieser Komponenten.

In marktüblichen Mehrkomponentengemischen, wie z.B. Schäumen, Schaum-, Dicht- und Klebstoffen, ist nach Herstellung des Gemisches bzw. nach Applikation des Gemisches eine Bestimmung der ursprünglichen Mischungsverhältnisse der Komponenten zueinander oftmals nicht möglich. Dies erschwert die qualitative Kontrolle des Herstellprozesses von Mehrkomponentengemischen und verhindert ein Nachvollziehen von Prozessabweichungen. In Folge dessen werden Gemische hergestellt, die die funktionellen Erwartungen nicht erfüllen können.

WO 2013/091623 A1 betrifft ein Verfahren zur Überwachung der Einhaltung einer vorgegebenen Sollkonzentration einer ersten Komponente in einem Artikel aus einem Thermoplast, der neben der ersten Komponente mindestens eine weitere Komponente enthält. Nach dem Präparieren einer Probe des Artikels erfolgt eine quantitative Analyse und Bestimmung der tatsächlichen Konzentrationen vorgegebener Elemente und/oder vorgegebener Verbindungen des Artikels, wobei die vorgegebenen Elemente ausgewählt sind aus den Elementen Strontium, Zirkonium und Zinn und die vorgegebenen Verbindungen ausgewählt sind aus Verbindungen der Elemente Strontium, Zirkonium und Zinn. Die Ist-Konzentrationen werden mit Soll-Konzentrationen verglichen und eine Einhaltung einer vorgegebenen Soll-Konzentration einer ersten Komponente wird überwacht.

US 4,478,941 A beschreibt das Bestimmen eines Verhältnisses von zwei oder mehr Komponenten, die zur Herstellung eines duroplastischen oder thermoplastischen Polymers verwendet werden, wobei das Bestimmen erfolgt, indem das resultierende Polymer analysiert wird, um das Verhältnis von Markierungselementen darin zu bestimmen, die separat in jede der zur Herstellung des Polymers verwendeten Komponenten eingebaut wurden.

DE 44 34 815 A1 betrifft einen Kunststoff, enthaltend mindestens einen Marker. Ferner beschrieben wird auch deren Herstellung und Nachweis sowie deren Verwendung bei der Herstellung von Halbzeugen oder Formteilen, insbesondere im Automobilbau, sowie für Dekorationszwecke in Wohn- und Gewerberäumen und zur Außendekoration.

EP 2 314 442 A1 offenbart Vernetzerbatche, umfassend eine markierende, pulverförmige Substanz mit einer Dichte größer 2 g/cm³. Das Herstellen des Vernetzerbatches umfasst das Mischen der Vernetzer mit Markierungsstoffen und ggf. zusätzlichen Bindemitteln, Stabilisatoren und/oder Füllstoffen und ggf. weiteren Hilfsstoffen.

Ausgehend von diesem Stand der Technik ist es eine Aufgabe der vorliegenden Erfindung ein Verfahren zur Bestimmung eines Mischungsverhältnisses von zwei organischen, mischbaren Komponenten in einem Gemisch dieser Komponenten anzugeben, das ohne hohen technischen und zeitlichen Aufwand umsetzbar ist und eine quantitative Analyse der für das Gemisch ursprünglich eingesetzten Komponentenmengen ermöglicht.

Die vorliegende Erfindung ist in den Ansprüchen definiert. Demnach wird die Aufgabe durch ein Verfahren zur Bestimmung eines Mischungsverhältnisses A von zwei organischen, mischbaren Komponenten in einem Gemisch dieser Komponenten gelöst, das nachfolgende Schritte umfasst: i) ein Bereitstellen von zwei organischen, mischbaren Komponenten in vordefinierten Mengen, ii) ein Vermischen eines anorganischen Markers mit einer der zwei Komponenten in einem vordefinierten Mischungsverhältnis von anorganischem Marker zu der einen der zwei organischen, mischbaren Komponenten, iii) ein Herstellen eines Gemisches der Komponenten, iv) ein Durchführen einer Analyse zur quantitativen Bestimmung der Menge des anorganischen Markers und v) ein Bestimmen des Mischungsverhältnisses A der zwei Komponenten aus der ermittelten Menge des anorganischen Markers über das vordefinierte Mischungsverhältnis des anorganischen Markers zur Komponente, wobei ein Anteil an anorganischem Marker, bezogen auf ein Gesamtgewicht des anorganischen Markers und der Komponente, der der anorganische Marker zugesetzt wird, 0,5 bis 2 Gew.-%, beträgt, wobei der anorganische Marker ZnS ist, und wobei die organischen Komponenten Ausgangsverbindungen von polymeren Kunststoffen sind.

Das erfindungsgemäße Verfahren findet Anwendung auf Gemische von unterschiedlichen organischen Komponenten, die insbesondere miteinander homogen mischbar sind, also ein im Wesentlichen einphasiges Gemisch bilden. Unter organischen Komponenten werden erfindungsgemäß solche chemischen Stoffe oder Stoffgemische verstanden, die funktionale, aktive oder reaktive Verbindungen sind oder enthalten, die im Wesentlichen auf Kohlenwasserstoffverbindungen basieren. Im Falle von Stoffgemischen können neben Kohlenwasserstoff-basierten Verbindungen auch andere organische oder anorganische Zusätze wie Füllstoffe, Pigmente, viskositätsregulierende Substanzen, Antioxidantien und der dergleichen, enthalten sein. Diese Zusätze beeinflussen die Funktionalität, Aktivität bzw. Reaktivität der Kohlenwasserstoff-basierten Verbindungen nicht. Ferner können die Komponenten auch funktionale Additive, wie z.B. Katalysatoren, enthalten. Die erfindungsgemäßen organischen Komponenten sind unter Ausgangsverbindungen von polymeren Kunststoffen zu finden.

Erfindungsgemäß wird eine Anzahl von zwei organischen, mischbaren Komponenten bereitgestellt. Eine Komponente wird mit einem bestimmten anorganischen Marker, nämlich ZnS, vermischt. Ein anorganischer Marker ist eine in Bezug auf die Komponenten und die Verarbeitung der Komponenten inerte, nicht flüchtige anorganische Verbindung, die keinen natürlichen Ursprung in den Komponenten aufweist. Ein Marker ist also eine anorganische Verbindung, die in den Komponenten nicht üblicherweise als Additiv enthalten ist. Da übliche Additive den organischen Komponenten oftmals in schwankenden Mengen (z.B. bei viskositätsregulierenden Substanzen) oder lediglich aus technischen Gründen zugesetzt werden, wie es z.B. bei Füllstoffen der Fall ist, oder aber unterschiedliche Komponenten dieselben Additive enthalten (z.B. dieselben Füllstoffe oder Pigmente), eignen sich herkömmlich zugesetzte Additive nicht als Marker im Sinne der Erfindung. Es wird diejenige

Komponente mit Marker versehen, deren Mischungsverhältnis A ermittelt werden soll. Wurde der anorganische Marker mit einer der Komponenten in einem vordefinierten Mischungsverhältnis von Marker zu Komponente vermischt, werden die Komponenten anschließend zu einem Gemisch verarbeitet. Dies kann mittels handelsüblicher Mischvorrichtungen, wie z.B. einem dynamischen Mischer, erfolgen. Das Gemisch kann auch Komponenten enthalten, die keinen Marker aufweisen, beispielsweise Hilfsstoffe, wie Lösungsmittel und anderweitige Additive. Ihre Mengen gehen nicht in das zu bestimmende Mischungsverhältnis A ein. Das Gemisch enthält damit homogen verteilt alle zugesetzten Komponenten und Additive sowie die Marker.

Im Anschluss daran erfolgt, beispielsweise nach Probenahme einer Probe aus dem Gemisch oder nach Weiterverarbeitung des Gemisches, beispielsweise nach Applikation des Gemisches, ein Durchführen einer Analyse zur quantitativen Bestimmung der Menge des anorganischen Markers. Das Gemisch wird also so analysiert, dass die Menge des Markers ermittelt wird. Sofern der Marker während der Analyse eine Reaktion eingeht, so wird die Analysetechnik so gewählt, dass die Stöchiometrie dieser Reaktion eindeutig bekannt ist, so dass auf die ursprüngliche Menge an Marker geschlossen werden kann.

Dadurch, dass das ursprüngliche Mischungsverhältnis des Markers zu der mit dem Marker vermischten Komponente bekannt ist, somit also ein vordefiniertes Mischungsverhältnis "Marker zu Komponente" vorliegt, kann, da der Marker inert und nicht flüchtig ist, durch die entsprechend ermittelte Menge an Marker auf die ursprüngliche Menge der Komponente geschlossen werden, woraus das Mischungsverhältnis A der Komponente ableitbar ist.

Das erfindungsgemäße Verfahren erfordert somit lediglich den Zusatz von quantitativ analysierbarem Marker zu üblicherweise verwendeten Komponenten sowie das quantitative Bestimmen des Markers in einem Gemisch der Komponenten. Die technischen sowie zeitlichen Anforderungen an das erfindungsgemäße Verfahren sind damit niedrig. Das Verfahren kann nach Probenahme parallel zur Produktion von Produkten aus dem Komponentengemisch durchgeführt werden, so dass Nachstellungen und Anpassungen der Produktion ermöglicht und ein hoher qualitativer Standard der hergestellten Produkte gewährleistet werden können. Das erfindungsgemäße Verfahren erlaubt eine einfache Kontrolle der Mischungsverhältnisse von Mehrkomponentensystemen zur Sicherung der Funktionalität und Qualität dieser Systeme.

Die Unteransprüche haben bevorzugte Ausgestaltungen und Weiterbildungen der Erfindung zum Inhalt.

Vorteilhaft wird hierbei diejenige Komponente nicht mit Marker versehen, die volumetrisch den größten Anteil am Gemisch der Komponenten ausmacht. Dies hat sich als vorteilhaft in Bezug auf die Messgenauigkeit herausgestellt.

Aufgrund der hohen chemischen Inertheit und Nichtflüchtigkeit, ist der anorganische Marker Zinksulfid (ZnS), auch, da sich die quantitative Analyse von ZnS einfach durchführen lässt und zu geringen Fehlerquoten führt. Des Weiteren ist ZnS in technischem Rahmen ausreichend verfügbar und nicht toxisch oder gesundheitsbedenklich.

Um Beeinträchtigungen der Komponenten hinsichtlich ihrer chemischen, physikalischen und mechanischen Eigenschaften weitgehend zu reduzieren, sowie auch um Kosten zu sparen, beträgt ein Anteil an anorganischem Marker, bezogen auf ein Gesamtgewicht aus dem anorganischen Marker und der Komponente, der der Marker zugesetzt wird, von 0,5 bis 2 Gew.-%.

Weiter vorteilhaft umfasst das Durchführen der Analyse eine Glührückstandsbestimmung des Gemisches gemäß DIN EN ISO 1172:1998-12 und/oder einen sauren Aufschluss des Gemisches. Eine Glührückstandsbestimmung erfolgt bei solchen Temperaturen, bei denen die funktionalen, aktiven oder reaktiven, im Wesentlichen auf Kohlenwasserstoffverbindungen basierenden Verbindungen der organischen Komponenten verbrennen und ausschließlich anorganische Produkte als Glührückstand zurückbleiben. Es wird bis zur Massekonstanz geglüht, so dass aus dem Masserückstand direkt auf das Mischungsverhältnis der Komponenten geschlossen werden kann. Ein saurer Aufschluss kann insbesondere mit Königswasser erfolgen. Hierdurch gehen alle üblichen anorganischen Marker in Lösung. Eine qualitative Analyse des Markers kann dann durch geeignete spektroskopische Verfahren erfolgen, wobei das spektroskopische Verfahren die Marker untereinander auch qualitativ unterscheidet.

Eine weitere vorteilhafte Weiterbildung sieht vor, dass das Durchführen der Analyse eine quantitative Bestimmung des anorganischen Markers mittels inductively-coupled-plasmaoptical-emission-spectrometry (ICP-OES), Atomabsorptionsspektroskopie (AAS) oder Atomemissionsspektroskopie (OES) umfasst. ICP-OES, auch bekannt als ICP-AES- oder ICP-Plasmaspektroskopie, ist eine optische Emissionsspektroskopie mit induktiv gekoppeltem Plasma (ICP) als Anregungsquelle, die eine einfache Handhabung, hohe Empfindlichkeit und Präzision sowie eine relative Freiheit von Interferenzen bietet. Sie ermöglicht gleichzeitig eine qualitative und quantitative Analyse des Markers, so dass der Komponente eine Menge an Marker zugeordnet werden kann.

Weiter vorteilhaft ist die organische, mischbare Komponente ausgewählt aus Ausgansverbindungen von Thermoplasten, Duroplasten und Elastomeren wie z.B. den verwendeten Monomeren. Diese organischen Verbindungen finden vielfach Anwendung in Mehrkomponentensystemen und funktionalen Produkten, so dass die Bestimmung des Mischungsverhältnisses ihrer Ausgangskomponenten hochrelevant ist. Vorzugsweise finden sich unter diesen organischen Komponenten, Ausgansverbindungen von insbesondere Polyurethane, Epoxide, Polyacrylate, Polyamide, Polyolefine und Mischungen daraus.

Ferner vorteilhaft findet das erfindungsgemäße Verfahren Anwendung auf ein Gemisch, das ein Klebstoff, Dichtstoff oder Schaumstoff ist. Gerade diese homogenen Gemische weisen eine hohe Funktionalität auf, deren Qualität durch Bestimmung des Mischungsverhältnisses A von wesentlicher Bedeutung ist.

Weitere Einzelheiten, Merkmale und Vorteile der Erfindung ergeben sich aus der nachfolgenden Beschreibung und der Figur. Es zeigt:

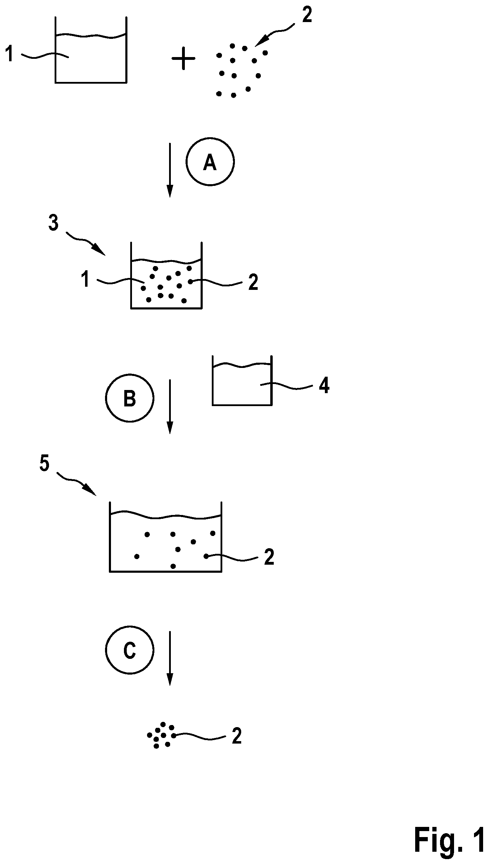

- Figur 1: einen schematischen Verfahrensablauf des erfindungsgemäßen Verfahrens gemäß einer vorteilhaften Weiterbildung.

Die vorliegende Erfindung wird anhand eines Ausführungsbeispiels im Detail erläutert. In Figur 1 sind nur die hier interessierenden Aspekte des erfindungsgemäßen Verfahrens dargestellt, alle übrigen Aspekte sind der Übersichtlichkeit halber weggelassen.

Im Detail zeigt Figur 1 die einfachste Ausgestaltung des erfindungsgemäßen Verfahrens zur Bestimmung eines Mischungsverhältnisses A von organischen, mischbaren Komponenten in einem Gemisch dieser Komponenten. Es wird die Herstellung eines Gemisches 5 aus zwei Komponenten 1, 4 beschrieben, wobei lediglich eine der Komponenten, nämlich eine erste Komponente 1, einen anorganischen Marker 2 umfasst. Zunächst wird die erste organische Komponente 1 bereitgestellt. Die erste organische Komponente ist eine Ausgangsverbindung eines Thermoplasts, eines Duroplasts oder eines Elastomers, beispielsweise ein Monomer. Die erste Komponente 1 wird in Schritt A mit einem anorganischen Marker 2 vermischt. Der anorganische Marker 2 ist ZnS, eine inerte, nicht flüchtige Verbindung, die weder mit der ersten Komponente 1 noch mit der später zuzufügenden zweiten Komponente 4 reagiert und auch nicht unter den Verarbeitungsbedingungen der Komponenten 1, 4 reagiert oder sich verflüchtigt. Der anorganische Marker 2 wird mit der ersten organischen Komponente 1 in einem bestimmten Mischungsverhältnis "Marker zu erster Komponente 1" gemischt. Dies bedeutet, dass die eingewogenen Mengen an erster organischer Komponente 1 und anorganischem Marker 2 bekannt sind. Es wird ein Gemisch 3 aus erster organischer Komponente 1 und anorganischem Marker 2 erhalten. Marker 2 und erste Komponente 1 sind in dem Gemisch homogen ineinander verteilt.

Separat wird die zweite Komponente 4 bereitgestellt. In Verfahrensschritt B erfolgt das Herstellen eines Gemisches 5 der ersten organischen Komponente 1 (mit Marker 2) und der zweiten organischen Komponente 4. Dadurch, dass die erste organische Komponente 1 und die zweite organische Komponente 4 miteinander mischbar sind, enthält das Gemisch 5 homogen verteilt die erste Komponente 1, die zweite Komponente 4 und den Marker 2.

In Verfahrensschritt C erfolgt das Durchführen einer Analyse zur quantitativen Bestimmung der Menge des anorganischen Markers 2 in dem Gemisch 5, beispielsweise nach Applikation des Gemisches 5. Dies kann z.B. durch Glührückstandsbestimmung des Gemisches 5 gemäß DIN ISO 1172: 1998-12 ausgeführt werden. Alternativ kann das Gemisch 5 sauer mit Königswasser aufgeschlossen und die erhaltene Lösung mit dem in Lösung gegangenen Marker 2 z.B. mittels ICP-OES analysiert und der Gehalt an Marker 2 quantitativ bestimmt werden. Aus der somit ermittelten Menge an Marker 2 kann über das vordefinierte Mischungsverhältnis "Marker 2 zu erster Komponente 1" auf die ursprüngliche Menge an erster organischer Komponente 1 und über das Gesamtgewicht des Gemisches 5 auf das Mischungsverhältnis A der ersten organischen Komponente 1 zu der zweiten organischen Komponente 4 geschlossen werden.

Die vorhergehende Beschreibung der vorliegenden Erfindung dient nur zu illustrativen Zwecken und nicht zum Zwecke der Beschränkung der Erfindung. Verschiedene Änderungen und Modifikationen sind innerhalb des durch die Ansprüche definierten Schutzbereichs möglich.

### Bezugszeichenliste:

- 1: erste organische Komponente
- 2: anorganischer Marker
- 3: Gemisch aus erster organischer Komponente und anorganischem Marker
- 4: zweiter organische Komponente
- 5: Gemisch aus erster organischer Komponente und zweiter organischer Komponente

### Bezugszeichenliste:

- 1: erste organische Komponente
- 2: anorganischer Marker
- 3: Gemisch aus erster organischer Komponente und anorganischem Marker
- 4: zweiter organische Komponente
- 5: Gemisch aus erster organischer Komponente und zweiter organischer Komponente

## Patentansprüche

1. Verfahren zur Bestimmung eines Mischungsverhältnisses A von 2 organischen, mischbaren Komponenten (1, 4) in einem Gemisch (5) enthaltend diese Komponenten, umfassend die Schritte:
- Bereitstellen der 2 organischen, mischbaren Komponenten (1, 4) in vordefinierten Mengen,
- Vermischen eines anorganischen Markers (2) mit einer der 2 organischen, mischbaren Komponenten (1) in einem vordefinierten Mischungsverhältnis von anorganischem Marker (2) zu der einen der 2 organischen, mischbaren Komponenten (1),
- Herstellen eines Gemisches (5) der Komponenten,
- Durchführen einer Analyse zur quantitativen Bestimmung der Menge des anorganischen Markers (2) und
- Bestimmen des Mischungsverhältnisses A der 2 organischen, mischbaren Komponenten (1, 4) unter Verwendung der ermittelten Menge des anorganischen Markers (2) über das vordefinierte Mischungsverhältnis,
wobei ein Anteil an anorganischem Marker (2), bezogen auf ein Gesamtgewicht des anorganischen Markers (2) und der Komponente (1), der der anorganische Marker (2) zugesetzt wird, 0,5 bis 2 Gew.-%, beträgt, **dadurch gekennzeichnet,**
**dass** der anorganische Marker (2) ZnS ist,
wobei die organischen Komponenten (1, 4) Ausgangsverbindungen von polymeren Kunststoffen sind.

2. Verfahren nach Anspruch 1, **dadurch gekennzeichnet, dass** das Durchführen der Analyse eine Glührückstandsbestimmung des Gemisches (5) und/oder einen sauren Aufschluss des Gemisches (5) umfasst.

3. Verfahren nach einem der vorhergehenden Ansprüche, **dadurch gekennzeichnet, dass** das Durchführen der Analyse eine quantitative Bestimmung des anorganischen Markers (2) mittels Atomabsorptionsspektroskopie oder Atomemissionsspektroskopie umfasst.

4. Verfahren nach einem der vorhergehenden Ansprüche, **dadurch gekennzeichnet, dass** die organischen, mischbaren Komponenten (1, 4) ausgewählt sind aus Ausgangsverbindungen von Thermoplasten, Duroplasten und Elastomeren, und insbesondere von Polyurethanen, Epoxiden, Polyimiden, Polyacrylaten, Polyamiden, Polyolefinen und Mischungen daraus.

5. Verfahren nach einem der vorhergehenden Ansprüche, **dadurch gekennzeichnet, dass** das Gemisch (5) ein Klebstoff, Dichtstoff oder Schaumstoff ist.

6. Verfahren nach einem der vorhergehenden Ansprüche, **dadurch gekennzeichnet, dass** das Gemisch (5) ferner Lösungsmittel und/oder Additive enthält.

## Claims

1. Method for determining a mixing ratio A of 2 organic miscible components (1, 4) in a mixture (5) comprising said components, comprising the steps of:
- providing the 2 organic miscible components (1, 4) in predefined amounts,
- mixing an inorganic marker (2) with one of the 2 organic miscible components (1) in a predefined mixing ratio of inorganic marker (2) to the one of the 2 organic miscible components (1),
- preparing a mixture (5) of the components,
- carrying out an analysis for the quantitative determination of the amount of the inorganic marker (2) and
- determining the mixing ratio A of the 2 organic miscible components (1, 4), using the amount of the inorganic marker (2) determined via the predefined mixing ratio,
wherein a proportion of inorganic marker (2), based on a total weight of the inorganic marker (2) and the component (1) added to the inorganic marker (2), is 0.5 to 2% by weight, **characterized in that**
the inorganic marker (2) is ZnS,
wherein the organic components (1, 4) are starting compounds of polymeric plastics.

2. Method according to Claim 1, **characterized in that** performing the analysis comprises determining the residue on ignition of the mixture (5) and/or an acid digestion of the mixture (5).

3. Method according to either of the preceding claims, **characterized in that** performing the analysis comprises quantitative determination of the inorganic marker (2) by means of atomic absorption spectroscopy or atomic emission spectroscopy.

4. Method according to any of the preceding claims, **characterized in that** the organic miscible components (1, 4) are selected from starting compounds of thermoplastics, thermosets and elastomers, and particularly of polyurethanes, epoxides, polyimides, polyacrylates, polyamides, polyolefins and mixtures thereof.

5. Method according to any of the preceding claims, **characterized in that** the mixture (5) is an adhesive, sealant or foam.

6. Method according to any of the preceding claims, **characterized in that** the mixture (5) further comprises solvents and/or additives.

## Revendications

1. Procédé de détermination d'un rapport de mélange A de 2 composants organiques miscibles (1, 4) dans un mélange (5) contenant ces composants, comprenant les étapes :
- fourniture de 2 composants organiques miscibles (1, 4) en des quantités prédéfinies,
- mélange d'un marqueur inorganique (2) avec l'un des composants organiques miscibles (1) selon un rapport de mélange prédéfini du marqueur inorganique (2) à l'un des 2 composants organiques miscibles (1),
- fabrication d'un mélange (5) des composants,
- mise en œuvre d'une analyse pour la détermination quantitative de la quantité du marqueur inorganique (2) et
- détermination du rapport de mélange A des 2 composants organiques miscibles (1, 4) par utilisation de la quantité déterminée du marqueur inorganique (2) à l'aide du rapport de mélange prédéfini,
dans lequel une proportion du marqueur inorganique (2), par rapport au poids total du marqueur inorganique (2) et du composant (1) auquel est ajouté le marqueur inorganique (2), est de 0,5 à 2 % en poids, **caractérisé en ce que**
le marqueur inorganique (2) est ZnS,
les composants organiques (1, 4) étant des composés de départ de plastiques polymères.

2. Procédé selon la revendication 1, **caractérisé en ce que** la mise en œuvre de l'analyse comprend une détermination du résidu d'incinération du mélange (5) et/ou une dissolution à l'acide du mélange (5).

3. Procédé selon l'une des revendications précédentes, **caractérisé en ce que** la mise en œuvre de l'analyse comprend une détermination quantitative du marqueur inorganique (2) par spectroscopie d'absorption atomique ou spectroscopie d'émission atomique.

4. Procédé selon l'une des revendications précédentes, **caractérisé en ce que** les composants organiques miscibles (1, 4) sont choisis parmi les composés de départ de thermoplastiques, de thermodurcissables et d'élastomères, et en particulier de polyuréthanes, d'époxydes, de polyimides, de polyacrylates, de polyamides, de polyoléfines et de mélanges de ceux-ci.

5. Procédé selon l'une des revendications précédentes, **caractérisé en ce que** le mélange (5) est une colle, un agent d'étanchéité ou une mousse.

6. Procédé selon l'une des revendications précédentes, **caractérisé en ce que** le mélange (5) contient en outre des solvants et/ou des additifs.
